(19) Europäisches Patentamt
European Patent Office
Office européen des brevets

(11) **EP 4 037 487 B1**

(12) **EUROPEAN PATENT SPECIFICATION**

(45) Date of publication and mention
of the grant of the patent:
**06.12.2023 Bulletin 2023/49**

(21) Application number: **20796689.6**

(22) Date of filing: **29.09.2020**

(51) International Patent Classification (IPC):
**A01N 59/16** (2006.01)    **A01N 31/02** (2006.01)
**A01N 25/04** (2006.01)    **A01N 25/10** (2006.01)
**A01N 25/34** (2006.01)    **A01P 1/00** (2006.01)

(52) Cooperative Patent Classification (CPC):
(C-Sets available)
**A01N 59/16**                                      (Cont.)

(86) International application number:
**PCT/CZ2020/000043**

(87) International publication number:
**WO 2021/063427 (08.04.2021 Gazette 2021/14)**

(54) **ANTIBACTERIAL SOL AND A METHOD FOR ITS PREPARATION**

ANTIBAKTERIELLES SOL UND EIN VERFAHREN ZU SEINER HERSTELLUNG

SOL ANTIBACTERIEN ET SON PROCÉDÉ DE PRÉPARATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.10.2019 CZ 20190612**

(43) Date of publication of application:
**10.08.2022 Bulletin 2022/32**

(73) Proprietor: **Grade Medical S.r.o.
27201 Kladno, Krocehlavy (CZ)**

(72) Inventors:
• **KAVALÍREK, Jan
15900 Praha 5, Velká Chuchle (CZ)**
• **BRATKA, Petr
14000 Praha 4, Krc (CZ)**

(74) Representative: **Skoda, Milan
Nahoranska 308
549 01 Nove Mesto nad Metuji (CZ)**

(56) References cited:
**CN-A- 101 368 330    CZ-U1- 31 200**

• **MUZALEV P A ET AL: "Synthesis, structure, and properties of silver nanocomposite materials with poly(hydroxyethyl methacrylate) matrix", RUSSIAN JOURNAL OF APPLIED CHEMISTRY, NAUKA/INTERPERIODICA, MO, vol. 84, no. 4, 19 May 2011 (2011-05-19), pages 666-669, XP019905239, ISSN: 1608-3296, DOI: 10.1134/S1070427211040185**
• **ZHIHAI CAO ET AL: "Synthesis of Silver/Poly(2-hydroxyethyl methacrylate) Particles via a Combination of Inverse Miniemulsion and Silver Ion Reduction in a "Nanoreactor"", LANGMUIR, vol. 27, no. 16, 16 August 2011 (2011-08-16), pages 9849-9859, XP55767967, US ISSN: 0743-7463, DOI: 10.1021/la202116s**
• **NEDA B. MALESIC ET AL: "Antimicrobial hydrogels based on 2-hydroxyethyl methacrylate and itaconic acid containing silver(I) ion", TEHNIKA / SAVEZ INZENJERA I TEHNICARA JUGOSLAVIJE, vol. 69, no. 4, 1 January 2014 (2014-01-01), pages 563-568, XP55767996, Serbia ISSN: 0040-2176, DOI: 10.5937/tehnika1404563M**

- **ANNA KEDZIORA ET AL: "Similarities and Differences between Silver Ions and Silver in Nanoforms as Antibacterial Agents", INTERNATIONAL JOURNAL OF MOLECULAR SCIENCES, vol. 19, no. 2, 2 February 2018 (2018-02-02), page 444, XP55767958, DOI: 10.3390/ijms19020444**
- **GIANLUIGI FRANCI ET AL: "Silver Nanoparticles as Potential Antibacterial Agents", MOLECULES, vol. 20, no. 5, 18 May 2015 (2015-05-18), pages 8856-8874, XP55375569, DE ISSN: 1433-1373, DOI: 10.3390/molecules20058856**
- **YI ZHANG ET AL: "Biocompatible and degradable poly(2-hydroxyethyl methacrylate) based polymers for biomedical applications", POLYMER CHEMISTRY, vol. 3, no. 10, 1 January 2012 (2012-01-01), pages 2752-2759, XP55767973, ISSN: 1759-9954, DOI: 10.1039/c2py20403g**
- **BALJIT SINGH ET AL: "Design of antibiotic drug loaded carbopol-cross-linked-poly(2-hydroxyethy methacrylate) hydrogel for wound dressing application", AMERICAN JOURNAL OF DRUG DELIVERY AND THERAPEUTICS, vol. 4, no. 1:2, 2 May 2017 (2017-05-02), pages 1-9, XP55768200,**
- **DI ZENG ET AL: "A transparent wound dressing based on bacterial cellulose whisker and poly(2-hydroxyethyl methacrylate)", ACTA PAEDIATRICA. SUPPLEMENT, vol. 105, 12 July 2017 (2017-07-12), pages 638-644, XP085237624, ISSN: 0141-8130, DOI: 10.1016/J.IJBIOMAC.2017.07.075**

(52) Cooperative Patent Classification (CPC): (Cont.)

C-Sets
**A01N 59/16, A01N 25/04, A01N 25/10, A01N 25/34, A01N 31/02**

## Description

## Technical Field

The invention relates to an antibacterial sol specifically to an antibacterial sol as a starting sol for the preparation of a silver-containing antibacterial layer, and to a method for its preparation.

## State of the Art

Antibacterial agents based on Ag compounds are known and are often used. For example, it is known how, from the file CZ 303861, to form an antibacterial layer by polymerisation, respectively how said layer is formed, created of a hybrid polymer of 3-(trimethoxysilyl) propyl methacrylate and titanium alkoxide with the addition of soluble salts of silver, copper and zinc and optionally also with the addition of titanium dioxide nanoparticles. Said composition is materially and technologically too complex so the object of the invention appears to be to discover the composition of the antibacterial layer and also the method of its preparation, where both the composition and the technology, and the method of preparation were simpler.

In more specific expression, it is apparent that a number of preparations organic-inorganic hybrid layers based on sol-gel have been described (e.g. Franc J. et al.: Mater. Sci. Eng. B129, 2006, p. 180) or the preparation of organic-inorganic hybrid nano-composites with $TiO_2$ and $SiO_2$ nanoparticles by sel-gel method with UV photopolymerisation of the organic network (Wang Z. et al.: Polym. Degrad. Stab. 91, 2006, p. 1455), where the authors focused on the study of mechanical properties and thermal stability, possibly photocatalytic properties. The antibacterial effects of the layers prepared from these q A-sols were not studied at all or were not significant. The antimicrobial properties of silver and its compounds, as mentioned above, have been well known for a long time. Silver metal and silver salts have been used as bactericides in burn dressings, polymer-bound silver salts, and as metallic silver layers (e.g., Atiyeh B.S. et al.: Burns 33, 2007, p. 139). However, certain limitations have been found, such as the interfering effect of the Ag salt, or the accumulation of metallic silver, or the inability to sustain a sufficiently effective concentration of Ag ions (Kim J. S. et al.: Mahomed. Nanotechnol. Biol. Med. 3, 2007, p. 95). Antibacterial layers of $SiO_2$-Ag prepared by the sel-gel method on glass (Hradecká H., Matoušek J.: Ceramics Silicates 54, 2010, p. 219) acted on E. Coli for 7 hours even without irradiation, but their preparation required a temperature of 500 up to 550°C, which precludes their application to plastics or finished medical devices with components that are not resistant to this temperature. Porous $TiO_2$-Ag composite layers (Necula BS et al.: Acta Biomaterialia 5, 2009, p. 3573) showed bactericidal activity against the bacterial strain MRSA, 100% inhibition of this strain was achieved in 24 hours. In contrast to patents CZ 303250 and CZ 305045, the antibacterial layer prepared according to this patent does not burden the environment with Zn and Cu salts and furthermore it is not necessary to crosslink it with heat for 1 to 3 hours after application to the substrate.

From patent document CZ 307398 an antibacterial salt is known as a starting material for the preparation of an antibacterial layer acting against pathogenic bacteria, especially S. gallinarium and E. coli, obtainable by dissolving trialkoxy ($C_1$-$C_4$) - silylpropyl methacrylate with the addition of soluble silver salt, preferably silver nitrate, the Ag content in dry matter being 0.1 to 5%, and a radical polymerisation initiator, preferably dibenzoyl peroxide, in ethanol or propan-2-ol, followed by the addition of an acid as a poly-condensation catalyst leading to the formation of an inorganic part of the network, with preferably nitric acid, and water, wherein the molar ratio of k = [$H_2O$]/[trialkoxy ($C_1$-$C_4$) -silylpropyl methacrylate] is in the range of 1.2 to 2.5 and the radical polymerisation of the double bonds trialkoxy ($C_1$-$C_4$)-silylpropyl methacrylate is brought to a conversion of 80 to 90%. The disadvantage of this sol is the high complexity of its preparation with the high sensitivity of the synthesis parameters on the output quality.

The patent document CN 101368330 A further discloses a method of preparing a polymer deoxidized nanosilver antibacterial finishing agent and its use, which is the application of this nanosilver antibacterial finishing agent to a fabric in order to prepare a ramie cotton fabric with antibacterial properties.

It follows from the above-noted current technology that its main disadvantage is the very complex and expensive production, which at the same time shows problems with the repeatability of the synthesis, with which the quality of its output is endangered.

The object of the invention is to formulate an antibacterial sol and to create a method for its preparation which is relatively simple and has a substantially lower sensitivity to the output quality on the synthesis conditions.

## Principle of the Invention

The above-mentioned disadvantages are largely eliminated and the objects of the invention are fulfilled by an antibacterial sol, specifically an antibacterial sol as a starting sol for the preparation of a silver-containing antibacterial layer, which according to the invention, is characterised by that as starting materials it contains 5.0 to 29.05 wt% 2-hydroxyethyl methacrylate, 0.05 to 0.25 wt% soluble silver salts, 0.005 to 0.1 wt% of silver nanoparticles (AgNPs), 0.1 to 0.6 wt% of radical polymerisation catalyst, and 70 to 90 wt% alcohol as a solvent. The advantage of this composition is especially its biocompatibility, because 2-hydroxyethyl methacrylate (HEMA), as an essential component of the emerging matrix of the antibacterial layer, is a polymer used for the manufacture of medical devices and the possibility of its use for medical applications while respectivly health safety is thus proven both in the literature and by a number of preclinical and clinical studies (eg Mokrý, Jaroslav & Karbanová, Jana & Lukas, Julius & Palecková, V & Dvoránková, B. (2000). CNS Diseases with Polymer-Encapsulated Cells. Biotechnology progress. 16. 897-904. 10.1021 / bp000113m.). Another advantage is the content of Ag in two forms, AgNOs and AgNPs, which brings a major advantage in terms of antibacterial effect over time, when the onset of bactericidal effects of silver cations is faster, moreover with diffusion outside the polymer matrix, while the effect of nanoparticles immobilised in the matrix is slower and basically bound to the surface of the matrix, but it can be considered stable.

To advantage, the antibacterial sol further contains 0.1 to 20 wt% water. Water shortens the time required to dissolve the primary materials, and thus increases the economic efficiency of the sol preparation and can also be used to influence the wet-ability of the resulting polymer matrix.

It is to great advantage if the silver nanoparticles (AgNPs) are nanoparticles prepared by the method of application of sodium citrate dihydrate, due to the relative simplicity of the synthesis and low sensitivity to its conditions, especially temperature, but also stability of synthesised AgNPs and the biocompatibility of used components. Of course, silver nanoparticles can also be produced by other production methods, for example by synthesis using microwave radiation.

It is to further great advantage if the silver nanoparticles (AgNPs) have an average size of 10 to 100 nanometers, at which the highest antibacterial activity is documented (e.g. Agnihotri S., Mukherji S. and Mukherji S.; Size-controlled silver nanoparticles synthesised over the range 5-100 nm using the same protocol and their antibacterial efficacy (RSC Adv., 2014, 4, 3974-3983) but with regard to the particle size acceptable for use to be in contact with the human organism.

It is also preferred that the soluble silver salt is silver nitrate, which is an approved and generally used substance for use in medical applications.

It is further preferred that the radical polymerisation catalyst is dibenzoyl peroxide (BPO).

The alcohol is most advantageously ethanol.

The above-mentioned disadvantages are largely eliminated and the objects of the invention are fulfilled by a method of production of antibacterial sol, with the above noted composition, which according to the invention is characterised by firstly dissolving 2-hydroxyethyl methacrylate and a radical polymerisation catalyst in alcohol, then adding a soluble silver salt to the resulting sol and boiling for 45 to 180 minutes, followed by the addition of silver nanoparticles (AgNPs) to the sol. The advantage is that this method of preparation is relatively simple and therefore relatively cheap as well. In addition, the synthesis of AgNPs alone allows for better control of the synthesis conditions and possibly also the control of the size of the stabilised nanoparticles before their being mixed with the other components.

To advantage, the silver nanoparticles (AgNPs) are prepared by adding aqueous solutions of AgNOs and $C_6H_7NaO_7$ to preheated $H_2O$. The advantage being that the Ag particles show a very small variance of their size depending on the fixed synthesis parameters.

Alternatively, it is advantageous if water is added to the sol at the same time as the soluble silver salt is added, which in particular improves its solubility or shortens the time required for complete dissolution.

It is advantageous to homogenise the distribution of silver nanoparticles (AgNPs) in the resulting sol, according to the first variant by means of ultrasound. Ultrasound can be applied to the sol without the need to insert a wave source into the solution, which does not increase the demands on the production process in terms of preventing contamination and its influence by contact of the sol with metal elements.

According to a second variant, the distribution of silver nanoparticles (AgNPs) in the antibacterial sol is simultaneously homogenised by stirring with a magnetic stirrer at a minimum speed of 600 rpm. This procedure is advantageous especially in the synthesis of overall higher volumes of sol (of the order of more than 5 litres), when the requirements for sufficiently powerful technology respecting technological equipment and operating conditions may be more economically advantageous in the case of using a magnetic stirrer.

The antibacterial salt thus prepared can advantageously be applied to various substrates, preferably to textile substrates, in order to treat them to be anti-bacterial. Application of antibacterial sol is realised mainly by spraying, conventional methods or preferably the method of so-called spray atomisation, further by using a foulard-for example the kiss-roll method, when the evaporation of solvents forms an antimicrobial layer, which does not require due to high conversion of antibacterial sol, reaching 70 - 90 %, further heat treatment, ie. completion of polymerisation (cross-linking). The resulting ratio of dry weight of antibacterial sol to weight of silver nanoparticles (AgNPs) is 99.9:0.1 to 25:75.

The main advantages of the antibacterial sol and its production method according to the invention as opposed to current technology, especially over the antibacterial sol known from patent document CZ 307398, are that the synergistic effect of silver ions and AgNPs is utilised and the addition of photoactive $TiO_2$ is eliminated as excess. The overall antibacterial effect in wound dressing conditions is considerably limited. Furthermore, Ag nanoparticles are synthesised separately with their subsequent addition, where the particles stabilised, e.g. by the method of sodium citrate dihydrate application, demonstrate significantly less scattering of these particles than the particles prepared by the sol-gel reaction, and the whole process thus shows significantly lower quality sensitivity output to synthesis conditions.

## Examples of the Performance of the Invention

Example 1

The antibacterial sol as a starting sol for the preparation of an antibacterial layer contains 14.5 wt% of 2-hydroxyethyl methacrylate, 0.15 wt% of a soluble silver salt, which is silver nitrate, 0.05 wt% of silver nanoparticles (AgNPs), 0.3 wt% of a radical polymerisation catalyst which is dibenzoyl peroxide (BPO), and 75 wt% alcohol as the solvent, which is ethanol. The antibacterial salt further contains 10 wt% water.

Silver nanoparticles (AgNPs) are nanoparticles which have an average size of 50 nanometers and which are prepared by the method of application of sodium citrate dihydrate.

According to the method of preparing the antibacterial sol, 2-hydroxyethyl methacrylate and the radical polymerisation catalyst are first dissolved in alcohol, then a soluble silver salt is added to the resulting sol and the whole is boiled for 90 minutes, followed by silver nanoparticles (AgNPs) being mixed into the sol.

Silver nanoparticles (AgNPs) are prepared by adding aqueous solutions of $AgNO_s$ and $C_6H_7NaO_7$ to preheated $H_2O$.

Simultaneously with the addition of the soluble silver salt, water is added to the sol.

The distribution of silver nanoparticles (AgNPs) in the antibacterial sol is simultaneously homogenised by using ultrasound.

The antibacterial sol thus prepared is applied to a textile substrate, whereby its antibacterial treatment is carried out.

The antibacterial effects of the layer thus prepared were tested in accordance with the generally used methodology for samples with non-leachable antibacterial treatment.

1. Bacterial culture - bacterial strain E. coli C600 was cultured in complete LB medium (Lysogenes broth, lysogenic broth). 100 $\mu$l of liquid culture was inoculated into 10 ml of LB medium and cultured stationary at 37°C overnight, i.e. about 16 hours.
2. Sample addition - From the initial bacterial culture grown overnight, a suspension was prepared into a micro-tube, type Eppendorf, 1 ml x number of samples. A portion of the solid sample (e.g., 1x1 cm in each 1 ml of suspension) was added to the cultures thus prepared.
3. Inoculation - the thus prepared bacterial cultures with added sample were left at 37°C for 24 hours in an incubator. After 24 hours, the samples were diluted to appropriate dilutions so that isolated colonies could be counted, and plated at 100 $\mu$l onto the surface of an agar plate. Inoculation was performed with a bent disposable rod on the entire surface of the agar plate. This is isolation by smearing to isolate a pure culture or to determine the number of microorganisms.
4. Incubation - Inoculated agar plates in petri dishes were placed in an incubator for 24 hours at 37°C.
5. Evaluation - after removal from the incubator, the grown live colonies were counted and the bactericidal effect of the sample was recalculated according to the respective control.

Preparation of agar, LB medium and saline for bacterial dilution

**Agar** - **LA Petri dishes**

- 10 g tryptone (Oxoid)

- 5 g yeast extract (Oxoid)

- 5 g NaCl (CarlRoth)

- 15 g agar technical NO 3 (Oxoid)

- quantity per 1000 ml of distilled water

- adjust the pH to 7.2 - 7.4

- sterilise in an autoclave 121 °C, 23 min

**LB medium** - **liquid**

- 10 g tryptone (Oxoid)

- 5 g yeast extract (Oxoid)

- 5 g NaCl (CarlRoth)

- quantity per 1000 ml of distilled water

- adjust the pH to 7.2 - 7.4

- sterilise in an autoclave 121 °C, 23 min.

**Phosphate saline**

- 9 g NaCl (CarlRoth)

- 1.2 g $Na_2HPO_4$ . 12 $H_2O$ (CarlRoth)

- 0.2 g $NaH_2PO_4$. 2 $H_2O$ (Penta)

- quantity per 1000 ml of distilled water

- adjust the pH to 7.2 - 7.4

- sterilise in an autoclave 121 °C, 23 min.

The evaluation was performed numerically, according to the formula:

Bactericidal effect (%) = A-B / A * 100

A - reference sample (control) - number of live bacteria
B - experimental sample - number of live bacteria

The HEMA I sample achieved 99.602% bactericidal activity as the worst result, the HEMA II sample achieved 99.597% bactericidal activity as the worst result, with the HEMA I and HEMA II samples differing with the addition of AgNOs.

Example 2

The antibacterial sol as a starting sol for the preparation of an antibacterial layer contains 5.0 wt% of 2-hydroxyethyl methacrylate, 0.05 wt% of a soluble silver salt, which is silver nitrate, 0.005 wt% of silver nanoparticles (AgNPs), 0.1 wt% of a radical polymerisation catalyst which is dibenzoyl peroxide (BPO), and 90 wt% alcohol as the solvent, which is ethanol. The antibacterial salt further comprises 4.845 wt% water.

Silver nanoparticles (AgNPs) are nanoparticles which have an average size of 10 nanometers and which are prepared by the method of application of sodium citrate dihydrate.

According to the method of preparing the antibacterial sol, 2-hydroxyethyl methacrylate and the radical polymerisation catalyst are first dissolved in alcohol, then a soluble silver salt is added to the resulting sol and the mixture is boiled for 45 minutes, followed by silver nanoparticles (AgNPs) being mixed into the sol.

Silver nanoparticles (AgNPs) are prepared by adding aqueous solutions of AgNOs and $C_6H_7NaO_7$ to preheated $H_2O$.

Simultaneously with the addition of the soluble silver salt, water is added to the sol.

The distribution of silver nanoparticles (AgNPs) in the antibacterial sol is simultaneously homogenised by stirring with a magnetic stirrer at a speed of at least 600 rpm. The antibacterial sol thus prepared is applied to a textile substrate, whereby its antibacterial treatment is carried out.

Example 3

The antibacterial sol as a starting sol for the preparation of an antibacterial layer contains 29.05 wt% of 2-hydroxyethyl methacrylate, 0.25 wt% of a soluble silver salt, which is silver nitrate, 0.1 wt% of silver nanoparticles (AgNPs), 0.6 wt% of a radical polymerisation catalyst which is dibenzoyl peroxide (BPO), and 70 wt% alcohol as the solvent, which is ethanol.

Silver nanoparticles (AgNPs) are two equal parts of nanoparticles, which have an average size of 40 and 100 nanometers, and which are prepared separately by the method of application of sodium citrate dihydrate.

According to the method of preparing the antibacterial sol, 2-hydroxyethyl methacrylate and the radical polymerisation catalyst are first dissolved in alcohol, then a soluble silver salt is added to the resulting sol and the mixture is boiled for 180 minutes, followed by silver nanoparticles (AgNPs) being mixed into the sol.

Silver nanoparticles (AgNPs) are prepared by adding aqueous solutions of AgNOs and $C_6H_7NaO_7$ to preheated $H_2O$.

The distribution of silver nanoparticles (AgNPs) in the antibacterial sol is simultaneously homogenised by using ultrasound.

The antibacterial sol thus prepared is applied to a textile substrate, whereby its antibacterial treatment is carried out.

Example 4

The antibacterial sol as a starting sol for the preparation of an antibacterial layer contains 17.5 wt% of 2-hydroxyethyl methacrylate, 0.15 wt% of a soluble silver salt, which is silver nitrate, 0.05 wt% of silver nanoparticles (AgNPs), 0.3 wt% of a radical polymerisation catalyst which is dibenzoyl peroxide (BPO), and 70 wt% alcohol as the solvent, which is ethanol. The antibacterial salt further contains 12 wt% water.

Silver nanoparticles (AgNPs) are nanoparticles which have an average size of 50 nanometers and which are prepared by the method of application of sodium citrate dihydrate.

According to the method of preparing the antibacterial sol, 2-hydroxyethyl methacrylate and the radical polymerisation catalyst are first dissolved in alcohol, then a soluble silver salt is added to the resulting sol and the mixture is boiled for 90 minutes.

Simultaneously with the addition of the soluble silver salt, water is added to the sol.

Silver nanoparticles (AgNPs) are prepared by adding aqueous solutions of AgNOs and $C_6H_7NaO_7$ to preheated $H_2O$.

The antibacterial sol base prepared as described above is applied to a textile substrate by a kiss-roll method, and then silver nanoparticles (AgNPs) are sprayed at intervals of up to 30 seconds to effect antibacterial treatment.

The advantage of this process is a higher ratio of particles immobilised on the surface of the resulting polymer matrix to particles that are completely or largely immobilised in the matrix mass, where they do not show any possibly minimal inhibitory activity.

## Industrial Application

The antibacterial sol, according to the invention is usable particularly in healthcare, for the preparation of mass-produced medical devices with an antibacterial (bactericidal, bacteriostatic) effect, specifically antibacterial wound dressings.

## Claims

1. An antibacterial sol, specifically an antibacterial sol as a starting sol for the preparation of an antibacterial layer containing silver, **characterised in that** it contains 5.0 to 29.05 wt% of 2-hydroxyethyl methacrylate, 0.05 to 0.25 wt% soluble silver salts, 0.005 to 0.1 wt% of silver nanoparticles (AgNPs), 0.1 to 0.6 wt% of radical polymerisation catalyst, and 70 to 90 wt% alcohol as a solvent.

2. The antibacterial sol according to claim 1, **characterised in that** it further comprises 0.1 to 20 wt% water.

3. The antibacterial sol according to any one of the preceding claims, **characterised in that** the silver nanoparticles (AgNPs) are nanoparticles prepared by the method of application of sodium citrate dihydrate.

4. The antibacterial sol according to any one of the preceding claims, **characterised in that** the silver nanoparticles (AgNPs) have an average size of from 10 to 100 nanometers.

5. The antibacterial sol according to any one of the preceding claims, **characterised in that** the soluble silver salt is silver nitrate.

6. The antibacterial sol according to any one of the preceding claims, **characterised in that** the radical polymerisation catalyst is dibenzoyl peroxide.

7. The antibacterial sol according to any one of the preceding claims, **characterised in that** the alcohol is ethanol.

8. A method for the preparation of an antibacterial sol according to any one of claims 1 to 7, **characterised in that** 2-hydroxyethyl methacrylate and a radical polymerisation catalyst are first dissolved in alcohol, then a soluble silver salt is added to the resulting sol and everything is boiled for 45 to 180 minutes, after which to the resulting sol, silver nanoparticles (AgNPs) are added.

9. The method for the production of the antibacterial sol, according to claim 8, **characterised in that** the silver nanoparticles (AgNPs) are prepared by adding aqueous solutions of AgNOs and sodium citrate dihydrate to the preheated $H_2O$.

10. The method for the preparation of the antibacterial sol according to claim 8, **characterised in that** at the same time as the soluble silver salt is added, water is added to the sol.

11. The method for the preparation of the antibacterial sol according to any one of claims 8 to 10, **characterised in that** the distribution of silver nanoparticles (AgNPs) in the antibacterial sol is simultaneously homogenised by means of ultrasound.

12. The method for the preparation of an antibacterial sol according to any one of claims 8 to 10, **characterised in that** the distribution of silver nanoparticles (AgNPs) in the antibacterial sol is simultaneously homogenised by stirring with a magnetic stirrer at a minimum speed of 600 rpm.

## Patentansprüche

1. Antibakterielles Sol, insbesondere ein antibakterielles Sol als Ausgangssol zur Herstellung einer antibakteriellen, silberhaltigen Schicht, **dadurch gekennzeichnet, dass** es 5,0 bis 29,05 Gew.-% 2-Hydroxyethylmethacrylat, 0,05 bis 0,25 Gew.-% lösliche Silbersalze, 0,005 bis 0,1 Gew.-% Silbernanopartikel (AgNP), 0,1 bis 0,6 Gew.-% radikalischen Polymerisationskatalysator und 70 bis 90 Gew.-% Alkohol als Lösungsmittel enthält.

2. Antibakterielles Sol nach Anspruch 1, **dadurch gekennzeichnet, dass** es außerdem 0,1 bis 20 Gew.-% Wasser enthält.

3. Antibakterielles Sol nach einem der vorhergehenden Ansprüche, **dadurch gekennzeichnet, dass** die Silbernanopartikel (AgNPs) Nanopartikel sind, die durch das Verfahren der Anwendung von Natriumcitratdihydrat hergestellt werden.

4. Antibakterielles Sol nach einem der vorhergehenden Ansprüche, **dadurch gekennzeichnet, dass** die Silber-Nanopartikel (AgNPs) eine durchschnittliche Größe von 10 bis 100 Nanometern aufweisen.

5. Antibakterielles Sol nach einem der vorangehenden Ansprüche, **dadurch gekennzeichnet, dass** das lösliche Silbersalz Silbernitrat ist.

6. Antibakterielles Sol nach einem der vorangehenden Ansprüche, **dadurch gekennzeichnet, dass** der Katalysator für die radikalische Polymerisation Dibenzoylperoxid ist.

**7.** Antibakterielles Sol nach einem der vorangehenden Ansprüche, **dadurch gekennzeichnet, dass** der Alkohol Ethanol ist.

**8.** Das Verfahren zur Herstellung des antibakteriellen Sols nach einem der Ansprüche 1 bis 7, **dadurch gekennzeichnet, dass** zunächst 2-Hydroxyethylmethacrylat und der Katalysator für die radikalische Polymerisation in Alkohol gelöst werden, anschließend ein lösliches Silbersalz zu dem resultierenden Sol zugegeben wird und das Ganze 45 bis 180 Minuten lang zum Sieden gebracht wird, wobei dem Sol danach Silbernanopartikel (AgNPs) zugesetzt werden.

**9.** Das Verfahren zur Herstellung des antibakteriellen Sols nach Anspruch 8, **dadurch gekennzeichnet, dass** die Silbernanopartikel (AgNPs) durch Zugabe von wässrigen Lösungen von AgNO3 und Natriumcitrat-Dihydrat zu dem vorgewärmten $H_2O$ hergestellt werden.

**10.** Das Verfahren zur Herstellung des antibakteriellen Sols nach Anspruch 8, **dadurch gekennzeichnet, dass** gleichzeitig mit der Zugabe des löslichen Silbersalzes dem Sol Wasser zugesetzt wird.

**11.** Das Verfahren zur Herstellung des antibakteriellen Sols nach einem der Ansprüche 8 bis 10, **dadurch gekennzeichnet, dass** die Verteilung der Silber-Nanopartikel (AgNPs) im antibakteriellen Sol gleichzeitig mit Ultraschall homogenisiert wird.

**12.** Das Verfahren zur Herstellung des antibakteriellen Sols nach einem der Ansprüche 8 bis 10, **dadurch gekennzeichnet, dass** die Verteilung der Silbernanopartikel (AgNPs) im antibakteriellen Sol gleichzeitig durch Rühren mit einem Magnetrührer bei einer Mindestgeschwindigkeit von 600 U/min homogenisiert wird.

**Revendications**

**1.** Un sol antibactérien, en particulier un sol antibactérien comme sol initial pour la préparation d'une couche antibactérienne contenant de l'argent, **caractérisé en ce qu'**il contient 5,0 à 29,05 % en poids de méthacrylate de 2-hydroxyéthyle, 0,05 à 0,25 % en poids de sel argenté soluble, 0,005 à 0,1 % en poids de nanoparticules d'argent (AgNP), 0,1 à 0,6 % en poids d'accélérateur de polymérisation radicalaire, et 70 à 90 % en poids d'alcool en tant que solvant.

**2.** Le sol antibactérien selon la revendication 1, **caractérisé en ce qu'**il contient également de 0,1 à 20 % en poids d'eau.

**3.** Sol antibactérien, selon l'une quelconque des revendications précédentes, **caractérisé en ce que** les nanoparticules d'argent (AgNP) sont des nanoparticules préparées par une méthode d'application de citrate de sodium dihydraté.

**4.** Sol antibactérien, selon l'une quelconque des revendications précédentes, **caractérisé en ce que** les nanoparticules d'argent (AgNP) ont une taille moyenne de 10 à 100 nanomètres.

**5.** Sol antibactérien, selon l'une quelconque des revendications précédentes, **caractérisé en ce que** le sel argenté soluble est le nitrate d'argent.

**6.** Sol antibactérien, selon l'une quelconque des revendications précédentes, **caractérisé en ce que** l'accélérateur de la polymérisation radicalaire est le peroxyde de dibenzoyle.

**7.** Sol antibactérien, selon l'une quelconque des revendications précédentes, **caractérisé en ce que** l'alcool est l'éthanol.

**8.** Méthode de préparation d'un sol antibactérien, selon l'une quelconque des revendications 1 à 7, **caractérisée en ce que** le méthacrylate de 2-hydroxyéthyle et l'accélérateur de polymérisation radicalaire sont d'abord dissous dans l'alcool, puis un sel argenté soluble est ajouté au sol obtenu et le tout est porté à ébullition pendant 45 à 180 minutes, des nanoparticules d'argent (AgNP) étant ensuite ajoutées au sol.

**9.** Méthode de préparation d'un sol antibactérien selon la revendication 8, **caractérisée en ce que** les nanoparticules d'argent (AgNP) sont préparées en ajoutant des solutions aqueuses d'AgNOs et de citrate de sodium dihydraté à de $H_2O$ préchauffé.

**10.** Méthode de préparation d'un sol antibactérien selon la revendication 8, **caractérisée en ce que** l'eau est ajoutée au sol en même temps que le sol argenté soluble.

**11.** Méthode de préparation d'un sol antibactérien selon l'une quelconque des revendications 8 à 10, **caractérisée en ce que** la distribution des nanoparticules d'argent (AgNP) dans le sol antibactérien est simultanément homogénéisée par ultrasons.

**12.** Méthode de préparation d'un sol antibactérien selon l'une quelconque des revendications 8 à 10, **caractérisée en ce que** la distribution des nanoparticules d'argent (AgNP) dans le sel antibactérien est simultanément homogénéisée par mélange à l'aide d'un agitateur magnétique à une vitesse d'au moins 600 tours par minute.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CZ 303250
- CZ 305045
- CZ 307398
- CN 101368330 A

### Non-patent literature cited in the description

- **FRANC J. et al.** *Mater. Sci. Eng.,* 2006, vol. B129, 180
- **WANG Z. et al.** *Polym. Degrad. Stab.,* 2006, vol. 91, 1455
- **ATIYEH B.S. et al.** *Burns,* 2007, vol. 33, 139
- **KIM J. S. et al.** *Mahomed. Nanotechnol. Biol. Med.,* 2007, vol. 3, 95
- **NECULA BS et al.** *Acta Biomaterialia,* 2009, vol. 5, 3573
- **MOKRÝ, JAROSLAV ; KARBANOVÁ, JANA ; LUKAS, JULIUS ; PALECKOVÁ, V ; DVORÁNKOVÁ, B.** CNS Diseases with Polymer-Encapsulated Cells. *Biotechnology progress.,* 2000, vol. 16, 897-904
- **AGNIHOTRI S. ; MUKHERJI S. ; MUKHERJI S.** Size-controlled silver nanoparticles synthesised over the range 5-100 nm using the same protocol and their antibacterial efficacy. *RSC Adv.,* 2014, vol. 4, 3974-3983